# EUROPÄISCHE PATENTSCHRIFT

(11) **EP 2 515 999 B1**
(45) Veröffentlichungstag und Bekanntmachung des Hinweises auf die Patenterteilung: **11.12.2024**
(21) Anmeldenummer: 10803237.6
(22) Anmeldetag: 22.12.2010
(51) Int. Cl.: A61N 2/02

(54) **VORRICHTUNG ZUR BEHANDLUNG MIT MAGNETISCHEN FELDERN**
TREATMENT DEVICE USING MAGNETIC FIELDS
DISPOSITIF DE TRAITEMENT PAR DES CHAMPS MAGNÉTIQUES

(30) Priorität: 23.12.2009 DE 102009060544
(43) Veröffentlichungstag der Anmeldung: 31.10.2012
(73) Patentinhaber: MedTec Medizintechnik GmbH, 35578 Wetzlar (DE)
(72) Erfinder: Muntermann, Axel, 35580 Wetzlar-Nauborn (DE)
(74) Vertreter: Blumbach · Zinngrebe Patentanwälte PartG mbB
(86) Internationale Anmeldenummer: PCT/EP2010/007856
(87) Internationale Veröffentlichungsnummer: WO 2011/076395

(56) Entgegenhaltungen:
- WO-A1-2005/075019
- DE-A1- 2 610 589
- GB-A- 134 640
- KERSCHNER B: "Schmerzlinderung durch Kernspinresonanz leere Behauptung", MEDIZIN-TRANSPARENT.AT - WISSEN WAS STIMMT, 21 August 2012 (2012-08-21), XP055277947, Retrieved from the Internet <URL:http://www.medizin-transparent.at/schmerzlinderung-durch-kernspinresonanz-leere-behauptung> [retrieved on 20160606]

## Beschreibung

### Gebiet der Erfindung

Die Erfindung betrifft eine Vorrichtung zur Behandlung mit magnetischen Feldern.

### Hintergrund der Erfindung

Verfahren zur Magnetfeldbehandlung sowie zugehörige Vorrichtungen zur Durchführung einer solchen sind bekannt.

Ein Verfahren zur Magnetfeldtherapie, bei welchem die therapeutische Wirkung auf dem Effekt der Kernspinresonanz beruht, ist in der europäischen Patentschrift EP 1 089 792 B1 (Patentinhaber Axel Muntermann) beschrieben.

Bei dem derartigen Verfahren wird eine Vorrichtung verwendet, bei welcher ein im Wesentlichen statisches Magnetfeld von einem senkrecht dazu stehenden magnetischen Wechselfeld überlagert wird.

Zur Erzielung einer Kernspinresonanz besteht die Anforderung, dass zumindest das statische Magnetfeld in der Behandlungszone eine möglichst homogene Feldstärke aufweist, da ansonsten eine Kernspinresonanz nur in einem Teilbereich der Behandlungszone erreicht werden kann.

Zur Erzeugung von magnetischen Feldern wird in der Praxis beispielsweise eine ringförmig ausgebildete Spule verwendet, die sich um den Patienten erstreckt.

Nachteilig an einer solchen Spulenanordnung oder an einer Ringspule im Allgemeinen ist, dass sich der Patient während der Behandlung in die Spule hineinlegen muss. Dies ist insbesondere für ältere oder fettleibige Menschen unangenehm, weiter kann die sich über den Patienten erstreckende ringförmige Anordnung Platzangst auslösen.

Weiter ist, insbesondere für gebrechliche Patienten, eine solche bekannte Vorrichtung schlecht zugänglich. So muss der Patient entweder selbst in die Ringspule klettern oder aufwändig auf einem Schlitten in die Ringspule geschoben werden.

In der DE2610589 ist eine Vorrichtung zur Magnetfeldbehandlung beschrieben, bei der sich Spulen zur Erzeugung der Magnetfelder in schwenkbaren Seitenteilen und in der Grundfläche befinden.

### Aufgabe der Erfindung

Der Erfindung liegt demgegenüber die Aufgabe zugrunde, die genannten Nachteile des Standes der Technik zumindest zu reduzieren.

Insbesondere ist es eine Aufgabe der Erfindung, eine Vorrichtung zur Magnetfeldtherapie bereitzustellen, deren Handhabbarkeit verbessert ist.

Eine weitere Aufgabe der Erfindung ist es, eine Vorrichtung zur Behandlung mit magnetischen Feldern bereit zu stellen, die eine große Behandlungszone aufweist.

### Zusammenfassung der Erfindung

Die Aufgabe der Erfindung wird bereits durch eine Vorrichtung zur Magnetfeldtherapie nach dem unabhängigen Anspruch gelöst.

Bevorzugte Ausführungsformen und Weiterbildung der Erfindung sind den jeweiligen Unteransprüchen zu entnehmen.

Die Erfindung betrifft eine Vorrichtung zur Behandlung mit magnetischen Feldern.

Die Vorrichtung umfasst eine vorzugsweise horizontal angeordnete Grundfläche sowie eine erste Einrichtung zum Erzeugen eines magnetischen Feldes. Die erste Einrichtung zum Erzeugen eines magnetischen Feldes ist vorzugsweise als Spule ausgebildet, mit welcher ein magnetisches Wechselfeld erzeugbar ist.

Weiter umfasst die Vorrichtung zwei sich vorzugsweise vertikale von den Seiten der Grundfläche aus erstreckende abgewinkelte Seitenteile, wobei die Seitenteile jeweils eine Spule aufweisen, deren Mittelachse quer, vorzugsweise im Wesentlichen senkrecht zum jeweiligen Seitenteil verläuft.

Über die sich gegenüberliegenden Spulen wird eine Helmholzspulenartige Anordnung geschaffen, die ein relativ homogenes Magnetfeld erzeugt, welches sich von einem Seitenteil zum anderen Seitenteil erstreckt.

Über die in den Seitenteilen angeordneten Spulen wird vorzugsweise ein im Wesentlichen statisches Magnetfeld erzeugt, wohingegen über die erste Einrichtung zum Erzeugen eines magnetischen Feldes ein senkrecht dazu stehendes Wechselfeld erzeugt wird.

Unter einem statischen Magnetfeld wird kein Magnetfeld verstanden, dessen Feldstärke sich überhaupt nicht ändert. Vielmehr ist gemäß einer bevorzugten Ausführungsform der Erfindung vorgesehen, das statische Feld mit einer geringeren Frequenz als das magnetische Wechselfeld zu sweepen. Die Feldstärke des statischen Feldes weist dabei einen Grundbetrag auf, der periodisch um einen gewissen Betrag erhöht oder erniedrigt wird. Über ein derartiges Sweepen kann sichergestellt werden, dass innerhalb der Behandlungszone die Resonanzbedingung erreicht wird. Ansonsten könnten bereits das Erdmagnetfeld oder magnetische Felder von elektrischen Geräten dazu führen, dass keine Resonanzbedingung im zu behandelnden Gewebe erreicht werden kann.

Die erste Einrichtung zum Erzeugen eines magnetischen Wechselfeldes umfasst eine in der Grundfläche angeordnete flach liegende Spule. Des Weiteren ist gemäß der Erfindung zumindest ein Seitenteil schwenkbar angeordnet. Über das gegenüber der Vorrichtung bewegliche Seitenteil wird erreicht, dass die Vorrichtung zur Behandlung mit magnetischen Feldern, welche, wie es bei einer bevorzugten Ausführungsform der Erfindung vorgesehen ist, eine Liege umfasst, von der Seite her frei zugänglich ist.

Dabei wird das Seitenteil, wie es erfindungsgemäß vorgesehen ist, im aufgestellten Zustand der Vorrichtung nach unten geschwenkt. So kann der Patient auch bei einer langgestreckten, als Liege ausgebildeten Vorrichtung auf bequeme Weise von der Seite die Liegefläche erreichen. Des Weiteren kann, sofern die Seitenteile hinreichend lang ausgebildet sind, eine Vorrichtung bereitgestellt werden, über die sich ein sehr großes Areal behandeln lässt.

Bei einer bevorzugten Ausführungsform der Erfindung ist die Vorrichtung als Liege ausgebildet, wobei sich die Seitenteile über zumindest 70 %, vorzugsweise über die gesamte Länge der Liegefläche erstrecken.

Erfindungsgemäß liegt die Schwenkachse für das eine schwenkbare Seitenteil im Wesentlichen des Bereichs des gegenüberliegenden Seitenteiles. Gegenüber einem Klappmechanismus, bei welchem das schwenkbare Seitenteil direkt an der Liege angeschlagen ist, hat die Erfindung den Vorteil, dass die Liegefläche nicht dauerhaft fest mit der restlichen Vorrichtung verbunden sein muss. So kann die Liege beispielsweise aus hygienischen Gründen auf einfache Weise ausgetauscht werden.

Ein weiterer Vorteil liegt darin begründet, dass durch den langen Hebel der durch den Anschlag des Seitenteils auf der gegenüberliegenden Seite bedingt ist, enge Biegeradien von Kabeln, mit welchen die im Seitenteil integrierte Spule mit Strom versorgt wird, vermieden werden.

Bei einer Weiterbildung der Erfindung sind die Seitenteile gekrümmt. Durch die gekrümmten Seitenteile wird das Raumgefühl verbessert. Im hochgeschwenkten Zustand erstrecken sich die Seitenteile bogenförmig um den Patienten, so dass diesem mehr Bewegungsfreiheit gelassen wird.

### Beschreibung der Zeichnungen

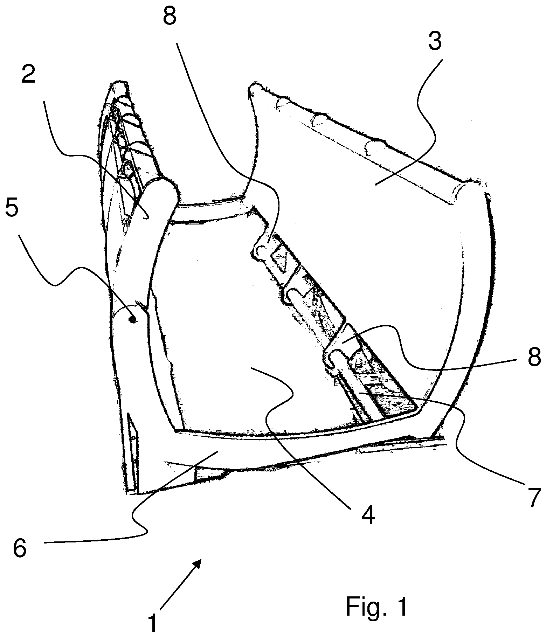

Die Erfindung soll im Folgenden anhand eines schematisch dargestellten Ausführungsbeispiels Bezug nehmend auf die Zeichnungen Fig. 1 bis Fig. 4 näher erläutert werden.

Fig. 1 zeigt ein schematisch dargestelltes Gerät zur Behandlung mit magnetischen Feldern 1.

Das Gerät zur Behandlung mit magnetischen Feldern 1 umfasst ein erstes Seitenteil 2 und ein zweites Seitenteil 3. Weiter 1 umfasst das Gerät zur Behandlung mit magnetischen Feldern eine Grundfläche 4, die beispielsweise als Liege für den Patienten ausgebildet ist. In dieser Darstellung ist die Liege, welche eingelegt werden kann, nicht dargestellt.

Die Seitenteile 2, 3 erstrecken sich im Wesentlichen über die gesamte Länge der Vorrichtung zur Behandlung mit magnetischen Feldern 1.

In den Seitenteilen 2, 3 sind Spanten 8 vorgesehen, welche als Träger für die Verkleidung, aber auch Träger für die in den Seitenteilen 2, 3 integrierten Spulen (nicht dargestellt) dienen.

Die Spanten 8 werden am unteren Ende durch jeweils eine Stange 7 zusammengehalten.

In dieser Vorrichtung zur Behandlung mit magnetischen Feldern 1 ist das rechte Seitenteil 3 schwenkbar ausgebildet. Dazu ist das Seitenteil 3 über einen Arm 6 mittels eines Gelenks 5 am linken Seitenteil 2 angeschlagen. Der Arm 6 ist im Wesentlichen L-förmig ausgebildet und in etwa mittig am Seitenteil 2 angeschlagen. So kann das Seitenteil 3 nach unten geschwenkt werden. Der relativ lange Arm 6 ermöglicht es, dass Biegeradien der Kabel, welche die im Seitenteil 3 integrierte Spule mit Strom versorgen, klein bleiben.

So kann auf eine Drehdurchführung zur Versorgung der Spule verzichtet werden.

Die sich zwischen Seitenteilen 2, 3 und Grundfläche 4 erstreckende Behandlungszone erfasst bei dieser Ausführungsform der Vorrichtung nahezu den gesamten Körper des Patienten. Im Unterschied zu bekannten Vorrichtungen, mit denen Kernspinresonanzen im zu behandelnden Gewebe erzielt werden, ist so eine Ganzkörperbehandlung möglich.

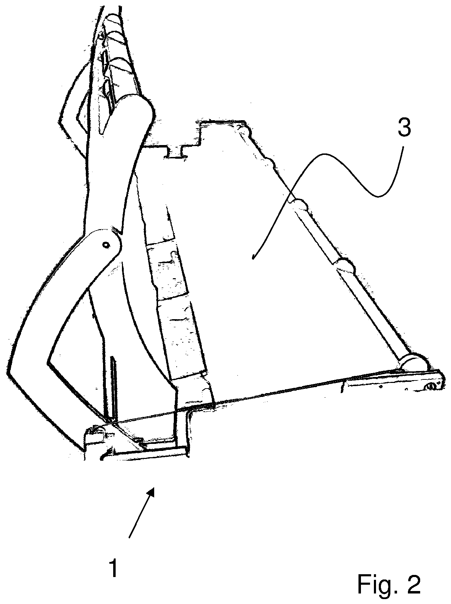

Fig. 2 zeigt die in Fig. 1 dargestellte Vorrichtung zur Behandlung mit magnetischen Feldern 1, wobei das rechte Seitenteil 3 heruntergeschwenkt ist. Es ist zu erkennen, dass die Vorrichtung zur Behandlung mit magnetischen Feldern nunmehr nicht mehr nur von den Stirnseiten und von oben her, sondern auch von der rechten Seite her frei zugänglich ist.

Bei heruntergeschwenktem Seitenteil 3 kann der Patient bequem auf einer Liege (nicht dargestellt) Platz nehmen und sich hinlegen. Sodann wird das Seitenteil 3 wieder hochgeschwenkt, um die Behandlung durchführen zu können.

Der Schwenkmechanismus kann sowohl motorisch als auch, bei einer alternativen Ausführungsform, zur manuellen Betätigung ausgebildet sein, beispielsweise mittels einer Handkurbel. Hierzu kann beispielsweise ein Seilzug oder eine Zahnstange vorgesehen sein, welche mittels eines Zahnrads, an dem eine Handkurbel oder ein Motor angebracht ist, bewegt wird.

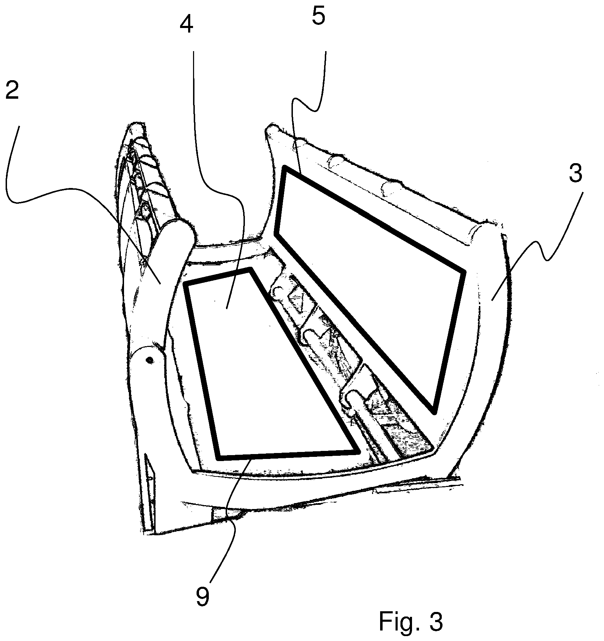

Bezug nehmend auf Fig. 3 ist schematisch die Anordnung der Spulen dargestellt.

In den Seitenteilen 2, 3 befindet sich jeweils eine Spule 5, wobei hier nur die Spule im rechten Seitenteil 3 dargestellt ist. Die Spule 5 folgt im Wesentlichen der Form des Seitenteils 3. Mit der im Seitenteil 2 integrierten Spule (nicht dargestellt) ergibt sich eine Helmholzspulenartige Anordnung, mittels der sich ein im Wesentlichen statisches Magnetfeld in der Behandlungszone erzeugen lässt. Es ist wichtig, dass das von den in den Seitenteilen 2, 3 integrierten Spulen erzeugte statische Feld eine möglichst gleichbleibende Feldstärke innerhalb der gesamten Behandlungszone aufweist. Um verbleibende Inhomogenitäten und äußere Einflüsse zu kompensieren, kann das von den Spulen in den Seitenteilen 2, 3 erzeugte statische Feld gesweept werden, so dass das Erreichen der Resonanzbedingung gewährleistet ist.

Zur Erzeugung eines im Wesentlichen senkrecht stehenden Wechselfeldes befindet sich oder unter der Grundfläche 4 eine Spule 9, deren Achse im Wesentlichen senkrecht zur Grundfläche 4 und senkrecht zur Spule 5 steht.

In diesem Ausführungsbeispiel folgt die Spule 9 auch im Wesentlichen der Form der Grundfläche 4, wobei die Spule 9 eine Vielzahl von Windungen umfasst. Bei einer alternativen Ausführungsform der Erfindung (nicht dargestellt) ist es aber auch denkbar, mehrere Spulen in oder unter der Grundfläche 4 unterzubringen. So ist beispielsweise auch die Verwendung von Spulen mit rundem Querschnitt vorgesehen. Mittels dieser Spulen kann überall innerhalb der Behandlungszone ein magnetisches Wechselfeld erzeugt werden.

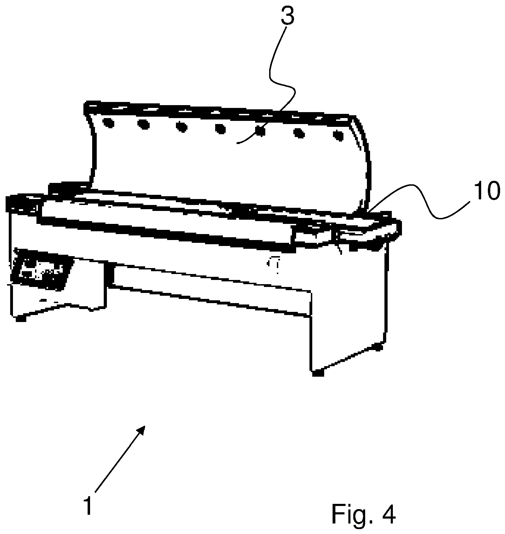

Fig. 4 zeigt, schematisch dargestellt, eine weitere, nicht erfindungsgemäße Variante.

Die Vorrichtung zur Behandlung mit magnetischen Feldern 1 umfasst bei diesem Beispiel ebenfalls eine Liege 10, auf welcher der Patient während der Behandlung ruhen kann.

Unterhalb der Liege ist eine Spule (nicht dargestellt) zur Erzeugung eines magnetischen Wechselfeldes angeordnet.

Weiter umfasst auch diese Vorrichtung zur Behandlung mit magnetischen Feldern 1 zwei Seitenteile, von denen das Seitenteil 3 zu sehen ist. Das weitere Seitenteil kann bei dieser Variante manuell oder motorisch in einen unterhalb der Liege 10 angeordneten Bereich bewegt werden, welcher vorzugsweise als Gehäuse ausgebildet ist. Im hier dargestellten Zustand ist das dem Seitenteil 3 gegenüberliegende Seitenteil im eingefahrenen Zustand dargestellt. Hierzu kann das Seitenteil beispielsweise über eine gekrümmte Zahnstange nach unter gefahren werden und dabei gleichzeitig in eine im Wesentlichen horizontale Position gebracht werden, sodass es unter das Gehäuse unter der Liege passt.

### Bezugszeichenliste

- 1: Vorrichtung zur Behandlung mit magnetischen Feldern
- 2: Seitenteil
- 3: Seitenteil
- 4: Grundfläche
- 5: Gelenk
- 6: Arm
- 7: Stange
- 8: Spanten
- 9: Spule
- 10: Liege
- 11: Lehne
- 12: Kopfapplikator
- 13: Drehgelenk

## Patentansprüche

1. Vorrichtung zur Behandlung mit magnetischen Feldern, umfassend
- eine horizontal angeordnete und als langgestreckte Liege ausgebildete Grundfläche, welche eine Länge definiert, mit entlang der Länge verlaufenden Längsseiten und mit Stirnseiten,
- eine erste Einrichtung zum Erzeugen eines magnetischen Feldes, welche zumindest eine in oder unter der Grundfläche angeordnete flach liegende Spule umfasst, und
- zwei sich vertikal von den Längsseiten der Grundfläche aus erstreckende gegenüberliegende Seitenteile, wobei die Seitenteile jeweils eine Spule aufweisen, deren Mittelachse senkrecht zum jeweiligen Seitenteil verläuft, und wobei zumindest ein Seitenteil nach unten schwenkbar an der Vorrichtung angebracht ist,
- **dadurch gekennzeichnet, dass**
- die Schwenkachse für das eine schwenkbare Seitenteil, welches über einen im Wesentlichen L-förmig ausgebildeten Arm mittels eines Gelenks am gegenüberliegenden Seitenteil angeschlagen ist, im Wesentlichen im Bereich des gegenüberliegenden Seitenteiles liegt, so dass die Liegefläche nicht dauerhaft fest mit der restlichen Vorrichtung verbunden ist.

2. Vorrichtung zur Behandlung mit magnetischen Feldern nach dem vorstehenden Anspruch, **dadurch gekennzeichnet, dass** das Seitenteil im aufgestellten Zustand der Vorrichtung nach unten schwenkbar ist.

3. Vorrichtung zur Behandlung mit magnetischen Feldern nach Anspruch 1, **dadurch gekennzeichnet, dass** zumindest ein Seitenteil seitlich, im Wesentlichen senkrecht zur Mittelachse zu seiner Spule schwenkbar ist.

4. Vorrichtung zur Behandlung mit magnetischen Feldern nach einem der vorstehenden Ansprüche, **dadurch gekennzeichnet, dass** die Vorrichtung derart ausgebildet ist, dass ein von den Spulen erzeugtes magnetisches Feld quer zu einem von der ersten Einrichtung zum Erzeugen eines magnetischen Feldes erzeugten magnetischen Feldes steht.

5. Vorrichtung zur Behandlung mit magnetischen Feldern nach einem der vorstehenden Ansprüche, **dadurch gekennzeichnet, dass** die Vorrichtung zur Magnetfeldtherapie gegenüber der Grundfläche offen ist.

6. Vorrichtung zur Behandlung mit magnetischen Feldern nach einem der vorstehenden Ansprüche, **dadurch gekennzeichnet, dass** die Seitenteile gekrümmt sind.

7. Vorrichtung zur Behandlung mit magnetischen Feldern nach einem der vorstehenden Ansprüche, **dadurch gekennzeichnet, dass** Vorrichtung als Liege ausgebildet ist, wobei sich die Seitenteile über zumindest 70%, vorzugsweise über die gesamte Länge der Liegefläche erstrecken.

8. Vorrichtung zur Behandlung mit magnetischen Feldern nach einem der vorstehenden Ansprüche, **dadurch gekennzeichnet, dass** über erste Einrichtung zur Erzeugung eines magnetischen Feldes ein magnetisches Wechselfeld erzeugbar ist und über die in den Seitenteilen angeordneten Spulen ein im Wesentlichen statisches Feld erzeugbar ist.

## Claims

1. Apparatus for treatment using magnetic fields, comprising
- a horizontally arranged base surface which is configured as an elongate couch and defines a length comprising longitudinal sides extending along the length and comprising end sides,
- a first device for generating a magnetic field, which comprises at least one coil that lies flat and is arranged in or under the base surface, and
- two opposing side parts which extend vertically from the base surface, wherein the side parts each comprise a coil, the central axis of which extends perpendicularly to the respective side part, and wherein at least one side part is attached to the apparatus so as to be pivotable downwards,
- **characterised in that**
- the pivot axis for one pivotable side part, which is fastened to the opposite side part via a substantially L-shaped arm by means of a joint, is located substantially in the region of the opposing side part, such that the lying surface is not permanently rigidly connected to the remainder of the apparatus.

2. Apparatus for treatment using magnetic fields according to the preceding claim, **characterised in that** the side part is pivotable downwards in the erected state of the apparatus.

3. Apparatus for treatment using magnetic fields according to claim 1, **characterised in that** at least one side part is pivotable laterally relative to its coil, substantially perpendicularly to the central axis.

4. Apparatus for treatment using magnetic fields according to any of the preceding claims, **characterised in that** the apparatus is configured such that a magnetic field generated by the coils is positioned transversely to a magnetic field generated by the first device for generating a magnetic field.

5. Apparatus for treatment using magnetic fields according to any of the preceding claims, **characterised in that** the apparatus is open opposite the base surface, for magnetic field therapy.

6. Apparatus for treatment using magnetic fields according to any of the preceding claims, **characterised in that** the side parts are curved.

7. Apparatus for treatment using magnetic fields according to any of the preceding claims, **characterised in that** the apparatus is configured as a couch, wherein the side parts extend over at least 70%, preferably over the entire length, of the lying surface.

8. Apparatus for treatment using magnetic fields according to any of the preceding claims, **characterised in that** an alternating magnetic field can be generated by the first device for generating a magnetic field, and a substantially static field can be generated by the coils arranged in the side parts.

## Revendications

1. Dispositif de traitement par des champs magnétiques, comprenant
- une surface de base disposée de manière horizontale et réalisée en tant que couchette allongée, laquelle définit une longueur, avec des côtés longitudinaux s'étendant le long de la longueur et avec des côtés frontaux,
- un premier système destiné à générer un champ magnétique, lequel comprend au moins une bobine située à plat disposée dans ou sous la surface de base, et
- deux parties latérales opposées s'étendant verticalement depuis les côtés longitudinaux de la surface de base, dans lequel les parties latérales présentent respectivement une bobine, dont l'axe central s'étend de manière perpendiculaire par rapport à la partie latérale respective, et dans lequel au moins une partie latérale est installée de manière à pouvoir pivoter vers le bas sur le dispositif,
- **caractérisé en ce que**
- l'axe de pivotement pour la partie latérale pouvant pivoter, laquelle est en butée contre la partie latérale opposée au moyen d'une articulation par l'intermédiaire d'un bras réalisé sensiblement en forme de L, se situe sensiblement dans la zone de la partie latérale opposée de telle sorte que la surface de couchette n'est pas reliée durablement et solidairement au dispositif restant.

2. Dispositif de traitement avec des champs magnétiques selon la revendication précédente, **caractérisé en ce que** la partie latérale peut être pivotée vers le bas dans l'état monté du dispositif.

3. Dispositif de traitement avec des champs magnétiques selon la revendication 1, **caractérisé en ce qu'**au moins une partie latérale peut être pivotée par rapport à une bobine latéralement, de manière sensiblement perpendiculaire par rapport à l'axe central.

4. Dispositif de traitement avec des champs magnétiques selon l'une quelconque des revendications précédentes, **caractérisé en ce que** le dispositif est réalisé de telle manière qu'un champ magnétique généré par les bobines est transversal par rapport à un champ magnétique généré par le premier système de génération d'un champ magnétique.

5. Dispositif de traitement avec des champs magnétiques selon l'une quelconque des revendications précédentes, **caractérisé en ce que** le dispositif est ouvert par rapport à la surface de base pour la thérapie par champ magnétique.

6. Dispositif de traitement avec des champs magnétiques selon l'une quelconque des revendications précédentes, **caractérisé en ce que** les parties latérales sont incurvées.

7. Dispositif de traitement avec des champs magnétiques selon l'une quelconque des revendications précédentes, **caractérisé en ce que** le dispositif est réalisé en tant que couchette, dans lequel les parties latérales s'étendent sur au moins 70 %, de préférence sur la totalité de la longueur de la surface de couchette.

8. Dispositif de traitement avec des champs magnétiques selon l'une quelconque des revendications précédentes, **caractérisé en ce qu'**un champ alternatif magnétique peut être généré par l'intermédiaire du premier système de génération d'un champ magnétique et un champ sensiblement statique peut être généré par l'intermédiaire des bobines disposées dans les parties latérales.
